# EUROPEAN PATENT SPECIFICATION

(11) **EP 4 561 665 B1**
(45) Date of publication and mention of the grant of the patent: **11.03.2026**
(21) Application number: 23785844.4
(22) Date of filing: 22.09.2023
(51) Int. Cl.: A61N 1/04, A61N 1/40, A61N 1/36

(54) **ELECTRODE ASSEMBLY WITH FILLER STRUCTURE BETWEEN ELECTRODE ELEMENTS**
ELEKTRODENANORDNUNG MIT FÜLLERSTRUKTUR ZWISCHEN ELEKTRODENELEMENTEN
ENSEMBLE ÉLECTRODE AVEC STRUCTURE DE REMPLISSAGE ENTRE DES ÉLÉMENTS D'ÉLECTRODE

(30) Priority: 22.09.2022 US 202263408918 P
(43) Date of publication of application: 04.06.2025
(73) Proprietor: NOVOCURE GMBH, 6340 Baar (CH)
(72) Inventor: WASSERMAN, Yoram, 31905 Haifa (IL); OBUCHOVSKY, Stas, 31905 Haifa (IL); KUPLENNIK, Nataliya, 31905 Haifa (IL); SHAPIRO, David, 31905 Haifa (IL); NAHSHONI, Avishai, 31905 Haifa (IL)
(74) Representative: Boden, Keith McMurray
(86) International application number: PCT/IB2023/059403
(87) International publication number: WO 2024/062449

(56) References cited:
- WO-A1-2008/006219
- JP-B2- 6 684 126
- US-A1- 2012 224 285
- US-A1- 2023 149 704

## Description

### BACKGROUND

Tumor Treating Fields (TTFields) therapy is a proven approach for treating tumors using alternating electric fields at frequencies between 50 kHz - 1 MHz, more commonly, 100-500 kHz. In current commercial systems, the alternating electric fields arc induced by electrode assemblies (e.g., arrays of capacitively coupled electrodes, also called transducer arrays) placed on opposite sides of the subject's body. When an AC voltage is applied between opposing electrode assemblies, an AC current is coupled through the electrode assemblies and into the subject's body. And higher currents are strongly correlated with higher efficacy of treatment.

WO-A-2023/089484, which is state of the art under Article 54(3) EPC, discloses electrode assemblies having at least two layers of conductive adhesive material separated by an anisotropic material.

US-A-2012/0224285 discloses conductive and dissipative electrostatic discharge (ESD) devices, and a method for manufacturing such devices, using conductive paths of low-cost particles in a curable non-conductive matrix.

JP-B-6684126 discloses an adhesive electrode and a measuring device which measures body surface potential.

WO-A-2008/006219 discloses a bio-electrode for conveying electrical signals to or from a body, which is constructed with two components - a pre-laminated member and a re-usable electrode assembly.

### SUMMARY

Disclosed herein, in one aspect, is an apparatus having an electrode layer according to claim 1.

Embodiments of the invention are disclosed in the dependent claims.

### BRIEF DESCRIPTION OF THE DRAWINGS

FIG. 1 is a top plan view of an electrode assembly in accordance with the present disclosure, with some layers shown as transparent in order to show underlying layers.
FIG. 2 is a cross-sectional view of the electrode assembly of FIG. 1, taken in the plane 2-2'. Dimensions are not shown to scale.
FIG. 3 is a close-up detail view of an exemplary electrode element of the cross section shown in FIG. 2.
FIG. 4 is a partial cross-sectional view of an exemplary electrode assembly in accordance with an embodiment disclosed herein, taken in the plane 2-2'. Dimensions are not shown to scale.
FIG. 5 is a schematic diagram of a system for providing tumor-treating fields as disclosed herein.

Various embodiments are described in detail below with reference to the accompanying drawings, wherein like reference numerals represent like elements, and wherein descriptions of like elements may not be repeated for every embodiment, but may be considered to be the same if previously described herein.

### DESCRIPTION OF THE PREFERRED EMBODIMENTS

This application describes exemplary electrode assemblies that may be used, e.g., for delivering TTFields to a subject's body and treating one or more cancers or tumors located in the subject's body.

The present invention can be understood more readily by reference to the following detailed description, examples, drawings, and claims, and their previous and following description. However, it is to be understood that this invention is not limited to the specific apparatuses, devices, systems, and/or methods disclosed unless otherwise specified, and as such, of course, can vary.

Headings are provided for convenience only and are not to be construed to limit the invention in any manner. Embodiments illustrated under any heading or in any portion of the disclosure may be combined with embodiments illustrated under the same or any other heading or other portion of the disclosure.

Any combination of the elements described herein in all possible variations thereof is encompassed by the invention unless otherwise indicated herein or otherwise clearly contradicted by context.

As used in the specification and the appended claims, the singular forms "a," "an" and "the" include plural referents unless the context clearly dictates otherwise. Thus, unless the context dictates otherwise, it is understood that disclosure of a singular form of an element can provide support for embodiments in which only a single one of such elements is provided, as well as for embodiments in which a plurality of such elements are provided.

In the preceding and following description, the terms "front," "inner," and "skin-facing" are used interchangeably to refer to a face or surface of the disclosed electrode assemblies (or components thereof) that faces or is oriented toward the skin of a subject (or generally toward the body of a subject) when used as disclosed herein. Similarly, the terms "rear," "upper," "outer," and "outwardly facing" are used interchangeably to refer to a face or surface of the disclosed electrode assemblies (or components thereof) that faces away from or is oriented away from the skin of a subject (or generally away from the body of the subject) when used as disclosed herein. For example, an "inner face" can face the skin of the subject, and an "outer face" can face away from the skin of the subject.

### Structure and Configuration of Apparatus

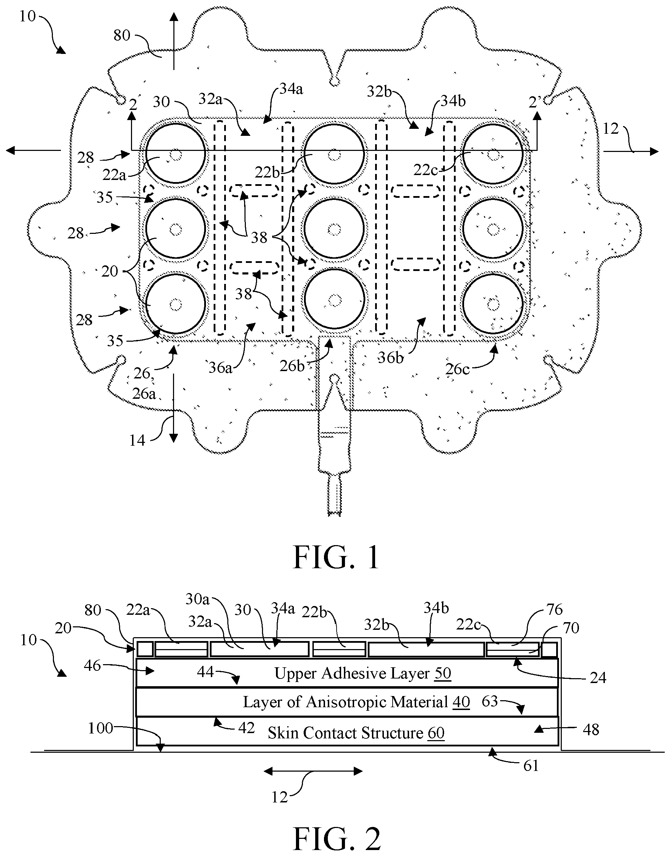

Referring to FIGS. 1-3, an apparatus 10 (also referred to herein as an "electrode assembly") can comprise an electrode layer 20 having a plurality of electrode elements 22 (e.g., 22a-c). Each electrode element 22 of the plurality of electrode elements can have a skin-facing surface 24. First and second electrode elements 22a,b can be spaced apart within the electrode layer 20 along a first axis 12. The electrode layer 20 can further comprise at least one filler structure 30. The at least one filler structure 30 can comprise a first filler structure 30a having a first filler portion 32a positioned within a space 34a between the first and second electrode elements 22a,b. The at least one filler structure 30 can be configured to provide the electrode layer 20 with a substantially consistent height along the first axis 12.

In some optional aspects, the apparatus 10 can further comprise a layer of anisotropic material 40 having a skin-facing surface 42 and an opposing outwardly facing surface 44 (FIG. 2). An upper adhesive layer 50 can comprise a conductive adhesive composite. The upper adhesive layer 50 can be disposed on an outer side 46 of the layer of anisotropic material. In some embodiments, the upper adhesive layer 50 is disposed on the outer side surface 44 of the layer of anisotropic material. The apparatus 10 can further comprise a skin contact structure 60 having at least one adhesive layer comprising a conductive adhesive composite. The skin contact structure can have an inner (skin facing) surface 61 and an outer side surface 63. The skin contact structure 60 can be disposed on an inner (skin-facing) side 48 of the layer of anisotropic material 40 and can be configured to contact skin 100 of a subject. In some embodiments, the skin contact structure 60 can be disposed on the inner (skin-facing) surface 42 of the layer of anisotropic material 40 and is configured to contact skin 100 of a subject. In some aspects, the electrode layer 20 can be on the outer side 46 of the layer of anisotropic material.

In other embodiments, the apparatus 10 can be free of a layer of anisotropic material (i.e., the apparatus 10 does not comprise a layer of anisotropic material). In these aspects, the upper adhesive layer 50 can be disposed on an outer side of the skin contact structure 60, or the upper adhesive layer 50 may also be absent in which case the skin contact structure can be disposed on an inner side of the electrode layer. Thus, the skin contact structure can be disposed on an inner side of the upper adhesive.

At least one electrode element 22 of the plurality of electrode elements can be in electrical contact with the outwardly facing surface 44 of the layer of anisotropic material 40.

In some aspects, each electrode element 22 of the plurality of electrode elements can be in electrical contact with the outwardly facing surface 44 of the layer of anisotropic material 40. Accordingly, the layer of anisotropic material 40 can extend continuously across an area within which each of the electrode elements is positioned.

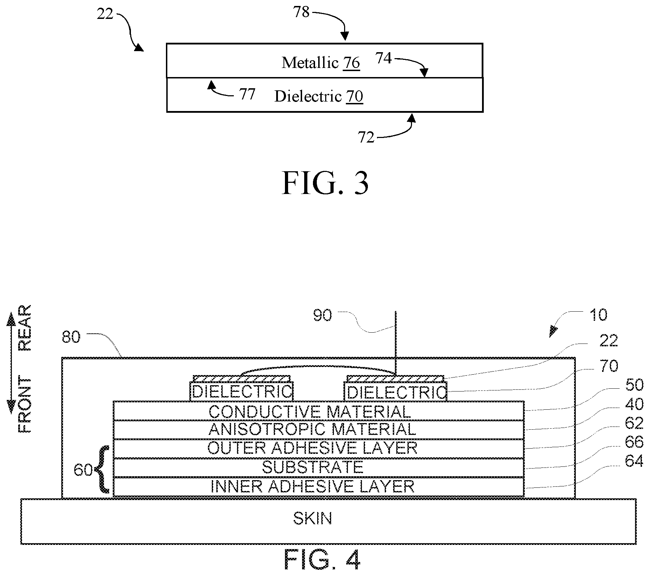

In some optional aspects, each electrode element 22 of the plurality of electrode elements can comprise a layer of dielectric material 70 having a skin-facing inner face 72 and an opposing outer face 74 (FIG. 3). In these aspects, the skin-facing inner face 72 of the dielectric material 70 can define the inner surface 24 of the electrode element 22. Each electrode element 22 can further comprise a metallic layer 76 having an inner face 77 and an opposing outer face 78. The metallic layer 76 can be disposed on the outer face 74 of the layer of dielectric material 70.

In some optional aspects, the dielectric material 70 can comprise a ceramic material. In other aspects, the dielectric material 70 can comprise a high dielectric polymer. In exemplary aspects, the dielectric material 70 can have a dielectric constant ranging from 10 to 50,000. In some embodiments, the layer of dielectric material 70 comprises a high dielectric polymer material such as poly(vinylidene fluoride-trifluoroethylenechlorotrifluoroethylene) and/or poly(vinylidene fluoride-trifluoroethylene-1-chlorofluoroethylene). Those two polymers are abbreviated herein as "Poly(VDF-TrFE-CTFE)" and "Poly(VDF-TrFE-CFE)," respectively. These embodiments are particularly advantageous because the dielectric constant of these materials is on the order of 40. In some embodiments, the polymer layer can be poly(vinylidene fluoride-trifluoroethylenechlorotrifluoroethylene-chlorofluoroethylene) or "Poly(VDF-TrFE-CTFE-CFE)." In some embodiments, the layer of dielectric material 70 comprises a terpolymer comprising polymerized units of monomers such as VDF, TrFE, CFE and/or CTFE in any suitable molar ratio. Suitable terpolymers include those, for example, having 30 to 80 mol% VDF, 5 to 60 mol% TrFE, with CFE and/or CTFE constituting the balance of the mol% of the terpolymer.

In alternative aspects, the electrode elements 22 do not comprise a dielectric material.

The plurality of electrode elements 22 can optionally comprise a third electrode element 22c. The second electrode element 22b can be positioned between, and spaced from, the first and third electrode elements 22a,c along, the first axis 12. The at least one filler structure 30 can further comprise a second filler portion 32b positioned between the second and third electrode elements 22b,c along the first axis 12. For example, the first filler structure 30a can have a second filler portion 32b positioned within a space 34b between the second and third electrode elements 22b,c. In other aspects, a second filler structure (not shown) that is not integrally formed with the first filler structure 30a can be positioned within the space 34b

In some optional aspects, the plurality of electrode elements 22 can comprise at least two rows 26 of electrode elements that extend along or parallel to a second axis 14 that is perpendicular to the first axis 12. The at least two rows of electrode elements can comprise a first row 26a of electrode elements 22 that includes the first electrode element 22a and a second row 26b of electrode elements that includes the second electrode element 22b. The first filler portion 32a can be positioned between the first and second rows 26a,b of electrode elements 22, for example, between electrode elements 22a and 22b along the first axis 12. Optionally, the plurality of electrode elements 22 can be arranged in exactly two rows 26, for example, as shown by row 26a and row 26b in isolation.

In further optional aspects, the plurality of electrode elements 22 can comprise at least three rows 26 (optionally, exactly three rows) of electrode elements 22 that extend along or parallel to a second axis 14 that is perpendicular to the first axis 12. The at least three rows 26 of electrode elements 22 can comprise a first row 26a of electrode elements that includes the first electrode element 22a, a second row of electrode elements 26b that includes the second electrode element 22b, and a third row of electrode elements 26c that includes the third electrode element 22c. The first filler portion 32a can be positioned between the first and second rows 26a,b of electrode elements 22, for example, between electrode elements 22a and 22b along the first axis 12. The second filler portion 32b can be positioned between the second and third rows 26b,c of electrode elements 22, for example, between electrode elements 22b and 22c along the first axis 12.

In various exemplary aspects, each row 26 of electrode elements 22 can comprise three electrode elements 22. In other aspects, each row 26 of electrode elements 22 can comprise two electrode elements 22, four electrode elements 22, five electrode elements 22, or more.

In some aspects, the at least one filler structure 30 can comprise foam. In some aspects, the at least one filler structure 30 can comprise any polymeric material, including rubber polymers, elastomers, thermoplastic polymers and thermosetting polymers. In some aspects, the at least one filler structure 30 can comprise a hydrocolloid. In some aspects, the at least one filler structure 30 can comprise a gel. Optionally, in these aspects, the at least one filler structure 30 can comprise a hydrocolloid gel. In various optional aspects, the filler structure can be or can comprise a medication.

The filler structure 30 can act as a medication substrate and the medication substrate may be capable of at least one of receiving, absorbing, or holding a topical medication applied thereto. Accordingly, the filler structure 30 can comprise a topical medication integrated in or on the filler structure. The topical medication can comprise a base component of oil, water, petrolatum, wax, cellulose, or a combination thereof. The topical medication can be a cream, an ointment, a lotion, a gel, a wax, a paste, or a mineral oil jelly. The topical medication can comprise at least one of an antibiotic, a steroid, an antiseptic, an emollient, an anesthetic, a terpene, a plant extract, a silicon-based organic polymer, an antifungal agent, a burn relief agent, a skin repair agent, an astringent, or an antihistamine. For example, the topical medication can be a steroid or corticosteroid, and can be a topical medication such as hydrocortisone or betamethasone. The topical medication can be or can comprise a skin repair agent that can include a hydrocolloid material. The topical medication can be any desired compound capable of soothing, healing, and/or providing relief for inflammation, sores, or other irritation that may develop on the skin of the subject's body. The topical medication can be substantially evenly distributed through a thickness of the medication substrate or, alternatively, the topical medication can be substantially disposed on the surface of the medication substrate.

When the filler structure is or comprises a hydrocolloid layer, the hydrocolloid layer can serve as a topical medication. The hydrocolloid layer can be a gel or it can include any suitable material or materials that can thicken when contacting a wound (e.g., based on absorbing liquid or secretions from damaged or wounded skin). The hydrocolloid layer can include any suitable material that can change into a gel or gel-like material when absorbing moisture from skin (e.g., hydrocolloid gel). In at least some exemplary embodiments, the hydrocolloid layer can include pectin, a material having collagen-like properties (e.g., gelatin), carboxymethyl cellulose (e.g., sodium carboxymethyl cellulose), and/or any other suitable material.

The hydrocolloid layer may or may not include adhesive and/or may or may not have adhesive properties. The hydrocolloid layer can include an adhesive layer similar to for example the adhesive layers disclosed herein. The hydrocolloid layer may act as an adhesive (e.g., have adhesive properties). Also for example, the hydrocolloid layer may neither include adhesive nor exhibit adhesive properties.

The medication can provide relief to the skin of the patient to counteract irritation caused by the apparatus 10. For example, for embodiments for which the filler material is or comprises a hydrocolloid, or for which the filler material is or comprises a medication, the electrode assembly may be moved (a translational movement) after a period of treatment so that the filler material now occupies a location such that it covers areas of the skin formerly covered by the electrodes. Performing such a translational movement of the electrode assembly serves the purpose of providing relief and some degree of healing to areas of the skin that may have suffered skin irritation due to the presence of the electrodes on the skin.

In some aspects, the at least one filler structure 30 and the plurality of electrode elements 22 have a combined surface area that is at least 20%, or at least 30%, or at least 40%, or at least 50%, or at least 60%, or at least 70% of a surface area of the layer of anisotropic material 40. In some aspects, the at least one filler structure 30 and the plurality of electrode elements 22 have a combined surface area that is no more than 80%, or no more than 70%, or no more than 60%, or no more than 50%, or no more than 40%, or no more than 30% of a surface area of the layer of anisotropic material 40.

In various aspects, the at least one filler structure 30, or portions thereof, can be complementary to the shapes of the electrode elements. In some optional aspects, the first filler portion and the second filler portion are part of a shaped layer of filler structure material that fills the gaps between all of the electrode elements, having cut-outs 35 as void spaces that receive respective electrode elements of the plurality of electrode elements. For example, in some optional aspects, the at least one filler structure 30 can be a single filler structure being a single contiguous structure that defines a plurality of cut-outs to respectively receive each electrode element of the plurality of electrode elements. The cut-outs 35 can be complementary to the shape of the electrode elements. It is further contemplated that the at least one filler structure 30 can be shaped to occupy all gaps between electrode elements arranged in any pattern, and not just in a rows-and-columns arrangement, whether as a single filler structure, or as a combination of multiple fitting filler portions.

In other aspects, the at least one filler structure 30 can comprise a plurality of separate elements. For example, a first filler structure can define the first filler portion 32, and a second filler structure can define the second filler portion 32 (for example, 32a and 32b).

In some aspects, the first filler portion 32a can have a shape that is complementary to a shape of the space 34a between the first and second electrode elements 22a,b.

In some optional aspects, the first filler portion 32a and/or the second filler portion 32b can be part of a strip of filler structure material that fills the gap between either one or more rows 26 of electrode elements or one or more columns 28 of electrode elements 22. For example, a first strip of filler material 36a can extend between the first and second rows 26a,b of electrode elements 22, and a second strip of filler material 36b can extend between the second and third rows 26b,c of electrode elements 22.

In further optional aspects, the filler structure 30 can comprise cut-outs 38 (shown in broken lines in FIG. 1) to promote flexibility and breathability of the apparatus 10. For example, the cut-outs 38 can include a single continuous cut-out or a plurality of cut-outs that are positioned along axes that extend between electrode elements 22 in order to promote flexibility between electrode elements. In some aspects, the cut-outs 38 can include a plurality of spaced cut-outs that extend along an axis positioned between columns 28 of electrode elements 22 or between rows 26 of electrode elements 22. In some aspects, the cut-outs 38 can comprise continuous cut-outs that extend continuously past a plurality of electrodes (e.g., along an entire row 26 of electrodes elements or along an entire column 28 of electrode elements). Optionally, the cut-outs 38 can comprise a plurality of cut-outs between sequential rows 26 of electrodes.

As discussed above, the upper adhesive layer 50 can be disposed on an outer side 46, or on the outer side surface 44, of the layer of anisotropic material. In some aspects, the upper adhesive layer 50 does not comprise hydrogel. For example, the upper adhesive layer 50 can comprise a conductive adhesive composite, as further disclosed herein. The conductive adhesive composite of the upper adhesive layer 50 can comprise a dielectric material and conductive particles dispersed within the dielectric material. Suitable dielectric materials include acrylic polymers and silicone polymers; and suitable conductive particles include carbon flakes, carbon granules, carbon fibers, carbon black powder, graphite powder, carbon nanotubes, carbon nanowires, and the like. For example, the upper adhesive layer 50 can comprise carbon black. In exemplary aspects, the upper adhesive layer 50 can comprise adhesive provided by ADHESIVE RESEARCH, such as ARcare^{®} 8006 electrically conductive adhesive composition manufactured and sold by Adhesives Research, Inc. (Glen Rock, PA, USA). In other optional aspects, the upper adhesive layer 50 can comprise carbon fibers or nanowires. For example, in exemplary aspects, the upper adhesive layer 50 can comprise a dry carbon/salt adhesive, such as the developmental product FLX068983 - FLEXcon^{®} OMNI-WAVETM TT 200 BLACK H-502 150 POLY H-9 44PP-8 from FLEXcon, Spencer, MA, USA, or other such OMNI-WAVE products from FLEXcon. In various aspects, the upper adhesive layer 50 can have a thickness from about 25 µm to about 150 µm.

In some optional aspects, the skin contact structure 60 can be a single layer comprising a conductive adhesive composite. In some aspects, the conductive adhesive composite comprises a dielectric material and conductive particles dispersed within the dielectric material, where suitable dielectric materials and suitable conductive particles are discussed above. In some aspects, the skin contact structure 60 can comprise an adhesive (e.g., acrylic adhesive) comprising carbon fiber or carbon nanowires. The skin contact structure 60 can comprise an adhesive (e.g., acrylic adhesive) comprising carbon black powder. In exemplary aspects, the skin contact structure 60 can have a thickness from about 25 µm to about 150 µm. For example, optionally, the skin contact structure 60 can have a thickness of about 50 µm. Although the conductive adhesive composite for the skin contact adhesive can have similar components, or even the same components, as the conductive adhesive composite for the upper adhesive layer, in any particular electrode assembly they may or may not be the same.

As illustrated in FIG. 4, in some optional aspects, the skin contact structure 60 can have an outer adhesive layer 62, an inner adhesive layer 64, and a substrate 66 positioned between the outer and inner adhesive layers 62, 64. Although shown in FIG. 2 as a single component, it is contemplated that the skin contact structure 60 depicted in FIG. 2 can include the inner and outer adhesive layers and substrate as shown in FIG. 4. The outer adhesive layer 62 can be disposed on a skin-facing side 48 of the layer of anisotropic material 40). Optionally, the outer adhesive layer 62 of the skin contact structure 60 can be disposed on the skin-facing surface 42 of the layer of anisotropic material. The inner adhesive layer 64 can be configured to contact skin of a subject. In further aspects, the inner adhesive layer 64 can be in contact with the skin of a subject.

In some aspects, the outer and inner adhesive layers 62, 64 of the skin contact structure 60 do not comprise hydrogel. For example, the outer and inner adhesive layers 62, 64 of the skin contact structure 60 can comprise a conductive adhesive composite, as further disclosed herein. The conductive adhesive composite of the inner and outer adhesive layers 64, 62 of the skin contact structure 60 can comprise a dielectric material and conductive particles dispersed within the dielectric material, as discussed above.

Alternatively, it is contemplated that the outer and/or inner adhesive layers 62, 64 can comprise hydrogel.

In some optional aspects, the outer adhesive layer 62 and/or the inner adhesive layer 64 can comprise carbon black. For example, in exemplary aspects, the outer adhesive layer 62 and/or the inner adhesive layer 64 can comprise adhesive provided by ADHESIVE RESEARCH, such as ARcare^{®} 8006 electrically conductive adhesive composition manufactured and sold by Adhesives Research, Inc. (Glen Rock, PA, USA). In other optional aspects, the outer adhesive layer 62 and/or the inner adhesive layer 64 can comprise carbon fibers or nanowires. For example, in exemplary aspects, the outer adhesive layer 62 and/or the inner adhesive layer 64 can comprise a dry carbon/salt adhesive, such as the developmental product FLX068983 - FLEXcon^{®} OMNI-WAVETM TT 200 BLACK H-502 150 POLY H-9 44PP-8 from FLEXcon, Spencer, MA, USA, or other such OMNI-WAVE products from FLEXcon.

In some optional aspects, the outer adhesive layer 62 can have a thickness of at least 40 µm (optionally, at least 45 µm or at least 50 µm). For example, the thickness of the outer adhesive layer 62 can range from about 40 µm to about 75 µm (e.g., optionally, from about 40 µm to about 70 µm, or from about 40 µm to about 65 µm, or from about 45 µm to about 75 µm, or from about 45 µm to about 70 µm, or from about 45 µm to about 65 µm or from about 50 µm to about 75 µm or from about 50 µm to about 70 µm, or from about 50 µm to about 65 µm). In further optional aspects, the inner adhesive layer 64 can have a thickness of at least 40 µm (optionally, at least 45 µm or at least 50 µm). For example, the thickness of the inner adhesive layer 64 can range from about 40 µm to about 75 µm (e.g., optionally, from about 40 µm to about 70 µm, or from about 40 µm to about 65 µm, or from about 45 µm to about 75 µm, or from about 45 µm to about 70 µm, or from about 45 µm to about 65 µm or from about 50 µm to about 75 µm or from about 50 µm to about 70 µm, or from about 50 µm to about 65 µm). It was discovered that an excessive thickness (e.g., greater than 75 µm or, even, greater than 65 µm in certain embodiments) can leave a residue following removal from a subject or patient. It was further discovered that an insufficient thickness (e.g., less than 25 µm in certain embodiments) can result in the layers breaking too easily.

In some embodiments, the substrate 66 of the skin contact structure 60 (FIG. 4) can be electrically conductive. In various optional aspects, the substrate 66 of the skin contact structure 60 can have a continuous, uninterrupted structure. In these aspects, it is contemplated that the substrate can be electrically conductive to conduct electricity between the outer and inner adhesive layers 62, 64, in like manner to scanning electron microscopy (SEM) tape.

In alternative aspects, the substrate 66 of the skin contact structure 60 can have an at least partially open structure that is configured to permit flow of adhesive between or among the inner and outer adhesive layers 64, 62 of the skin contact structure. In this way, the adhesive can conduct electricity through the substrate 66. For example, in some aspects, the substrate 66 can comprise a mesh. Optionally, the mesh can have a density from about 6 grams per square meter to about 8 grams per square meter. In other aspects, the substrate 66 can comprise a scrim.

In various optional aspects, the substrate 66 of the skin contact structure 60 can comprise paper, any suitable polymer (e.g., polyester, polyolefin, etc.), or fabric.

Optionally, the skin contact structure 60 can be reusable.

In some optional aspects, the upper adhesive layer 50 does not comprise hydrogel. In further or alternative aspects, the skin contact structure 60 does not comprise hydrogel. Accordingly, in some optional aspects, both the upper adhesive layer 50 and the skin contact structure 60 do not comprise hydrogel. In other aspects, either the upper adhesive layer 50 or the skin contact structure 60, or both, may comprise hydrogel.

In exemplary aspects, the conductive adhesive composite of any of the layers of the apparatus can comprise a dielectric material and conductive particles dispersed within the dielectric material. In some embodiments, at least a portion of the conductive particles define a conductive pathway through a thickness of the conductive adhesive composite. It is contemplated that the conductive particles can be aligned in response to application of an electric field such that the conductive particles undergo electrophoresis. In some aspects, the dielectric material of the conductive adhesive composite of each of the first and second electrode assemblies is a polymeric adhesive. Optionally, in these aspects, the polymeric adhesive can be an acrylic adhesive. In some aspects, the conductive particles can comprise carbon. Optionally, in these aspects, the conductive particles can comprise graphite powder. Additionally, or alternatively, the conductive particles can comprise carbon flakes. Additionally, or alternatively, the conductive particles can comprise carbon granules. Additionally, or alternatively, the conductive particles can comprise carbon fibers. Additionally, or alternatively, the conductive particles can comprise carbon nanotubes or carbon nanowires. Additionally, or alternatively, the conductive particles can comprise carbon black powder. In further aspects, the conductive adhesive composite further comprises a polar material (e.g., a polar salt). The polar salt may be a quaternary ammonium salt, such as a tetra alkyl ammonium salt. Exemplary conductive adhesive composites, as well as methods for making such conductive adhesive composites, are disclosed in U.S. Patent No. 8,673,184 and U.S. Patent No. 9,947,432, which are incorporated herein by reference for all purposes. In exemplary aspects, the conductive adhesive composite can be a dry carbon/salt adhesive, such as the OMNI-WAVE adhesive compositions, described above, manufactured and sold by FLEXCON (Spencer, MA, USA).

In some aspects, the layer of anisotropic material 40 has a first thermal conductivity in a direction that is perpendicular to a plane of the layer. The thermal conductivity of the layer of anisotropic material 40 in directions that are parallel to the plane of the layer of anisotropic material can be more than two times higher than the first thermal conductivity. For example, in some aspects, the thermal conductivity of the layer of anisotropic material 40 in directions that are parallel to the plane of the layer of anisotropic material can be more than three times higher, more than four times higher, or more five times higher than the first thermal conductivity. In some aspects, the thermal conductivity in the parallel directions is more than ten times higher than the first thermal conductivity. In various aspects, for example, the thermal conductivity of the layer of isotropic material 40 in directions that are parallel to the plane of the layer of anisotropic material can be more than: 1.5 times, 2 times, 3 times, 5 times, 10 times, 20 times, 100 times, 200 times, or even more than 1,000 times higher than the first thermal conductivity.

The layer of anisotropic material 40 can have a first resistance in a direction that is perpendicular to a plane of the layer. In some optional aspects, resistance of the layer in directions that are parallel to the plane of the layer is less than half the first resistance. In exemplary aspects, the resistance of the layer of anisotropic material 40 in directions that are parallel to the plane of the layer can be less than 10% of the first resistance. In exemplary aspects, the resistance of the layer of anisotropic material 40 in directions that are parallel to the plane of the layer can be less than 75%, 50%, 40%, 30%, 20%, 10%, 5%, 1%, 0.5%, or even less than 0.1% of the first.

In some optional aspects, the anisotropic material can comprise graphite. In some optional aspects, the graphite can comprise synthetic graphite. The layer of anisotropic material can be, or can comprise, a layer of pyrolytic graphite, graphitized polymer film, or graphite foil made from compressed high purity exfoliated mineral graphite. Examples of suitable forms of graphite include synthetic graphite, such as pyrolytic graphite (including, but not limited to, Pyrolytic Graphite Sheet (PGS), available from Panasonic Industry, Kadoma, Osaka, Japan), other forms of synthetic graphite, including but not limited to, graphite foil made from compressed high purity exfoliated mineral graphite (including, but not limited to, that supplied by MinGraph^{®} 2010A Flexible Graphite, available from Mineral Seal Corp., Tucson, Arizona, USA), or graphitized polymer film, e.g., graphitized polyimide film, (including, but not limited to, that supplied by Kaneka Corp., Moka, Tochigi, Japan. In alternative embodiments, conductive anisotropic materials other than graphite may be used instead of graphite.

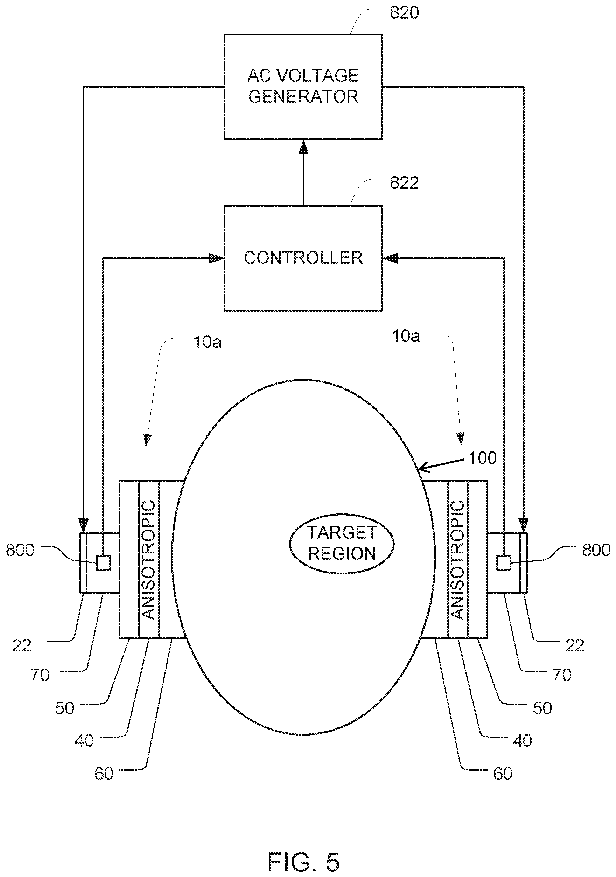

The plurality of electrode clements 22, can be wired together (e.g., using wires, traces on a flex circuit, etc.) to a lead 90 (FIG. 4). The lead 90 can supply an AC voltage from an AC voltage generator 820 (FIG. 5) to the electrode elements to generate the TTFields when the apparatus 10 (for example 10a and 10b in FIG. 5) is affixed to the subject's body for treatment.

The apparatus 10 comprises a flexible self-adhesive backing 80 (FIGS. 1, 2 and 4) that overlies the outer side of at least a portion (optionally all) of the layer of anisotropic material 40. The flexible self-adhesive backing 80 can promote adhesion of the apparatus to the skin of the patient as well as provide stability and support to the apparatus 10. In a conventional electrode assembly, individual electrode elements are positioned against, and contact, the skin of the patient via a layer of conductive hydrogel. An alternating current may be applied to the patient's body through the skin of the patient via individual electrode elements, which induces an electric field within the body. Conventionally, the electrode elements include ceramic disks as the dielectric layer and these result in a raised profile at each of the electrodes (and an uneven depth profile across the whole area of the electrode assembly). As disclosed herein a continuous conductive structure provided by the layer of conductive anisotropic material (such as a sheet of pyrolytic graphite) can extend across a plurality of electrode elements (optionally, all of the electrode elements). Further, the filler structure can even out the surface of the continuous conductive structure, rather than forming ridges and valleys in between rows/columns of electrode elements. The combination of the continuous conductive structure and the filler structure provides better conduction than "perimeter isolation" that is associated with conventional electrode assemblies, in which conduction is provided only along the z-axis and not in the x-y plane, along which the array extends. Further, the filler structure 30 can provide support to the apparatus 10 to improve performance of the apparatus. Omitting the filler structure, thereby leaving large gaps between the electrode elements, can significantly weaken the structural support of the apparatus, which, in turn, can cause imperfect contact between an electrode and the skin which reduces conduction across the apparatus and can cause hot spots. Further, as discussed above, for embodiments for which the filler material is or comprises a hydrocolloid, or for which the filler material is or comprises a medication, the electrode assembly may be moved (a translational movement) after a period of treatment so that the filler material now occupies a location such that it covers areas of the skin formerly covered by the electrodes. Performing such a translational movement of the electrode assembly serves the purpose of providing relief and some degree of healing to areas of the skin that may have suffered skin irritation due to the presence of the electrodes on the skin. Omitting the filler structure comprising a hydrocolloid or a medication would fail to provide medicated relief, but even if a medication were utilized without the filler material, there would be large gaps in the areas between the electrode elements due to the uneven depth profile across the area of the electrode assembly, and contact to the skin would be minimal or at least incomplete.

### Method of Use of Apparatuses

Referring also to FIG.5, a method can comprise positioning at least first and second electrode assemblies 10a,b on a body of a subject. Each of the first and second electrode assemblies 10a,b can be an apparatus 10 as described herein. For example, each of the first and second electrode assemblies 10a,b can comprise an electrode layer 20 (FIGS. 1-2) having a plurality of electrode elements 22.

As discussed above and with reference to FIGS. 1-4, each electrode element 22 of the plurality of electrode elements can have having a skin-facing surface 24. First and second electrode elements 22a,b can be spaced apart within the electrode layer 20 along a first axis 12. The electrode layer 20 can further comprise at least one filler structure 30. The at least one filler structure 30 can comprise a first filler structure 30a having a first filler portion 32a positioned within a space 34a between the first and second electrode elements. The at least one filler structure 30 can be configured to provide the electrode layer 20 with a substantially consistent height along the first axis 12.

The first and second electrode assemblies 10a,b can each further comprise a layer of anisotropic material 40 having a skin-facing surface 42 and an opposing outwardly facing surface 44. An upper adhesive layer 50 can comprise a conductive adhesive composite. The upper adhesive layer 50 can be disposed on an outer side 46 of the layer of anisotropic material. The apparatus 10 can comprise a skin contact structure 60 having an adhesive layer comprising a conductive adhesive composite. The skin contact structure 60 can be disposed on an inner (skin-facing) side 48 of the layer of anisotropic material 40, or it can be disposed directly on the inner (skin-facing) surface 42 of the layer of anisotropic material 40. The skin contact structure 60 can be a single layer adhesive (a conductive adhesive composite), or, alternatively, the skin contact structure 60 may comprise multiple layers. For example, the skin contact structure 60 may comprise an outer adhesive layer 62, an inner adhesive layer 64, and a substrate 66 positioned between the outer and inner adhesive layers 62, 64, as discussed above.

At least one electrode element 22 of the plurality of electrode elements can be in electrical contact with the outwardly facing surface 44 of the layer of anisotropic material 40.

The skin contact structure 60 of each of the first and second electrode assemblies 10a,b can contact skin 100 of the subject. An alternating voltage can be applied between the first electrode assembly 10a and the second electrode assembly 10b, thereby generating an electric field.

The alternating voltage between the first electrode assembly and the second electrode assembly 10a,b can be applied by an AC voltage generator 820. In some embodiments, the frequency of the alternating voltage is between 50 kHz and 1 MHz, or between 100 kHz and 500 kHz. In the illustrated example, the AC voltage generator is controlled by a controller 822. The controller 822 may use temperature measurements to control the amplitude of the current to be delivered via the first and second electrode assemblies 10a and 10b in order to maintain temperatures below a safety threshold (e.g., 41° C). This may be accomplished, for example, by measuring a first temperature of the first electrode element, measuring a second temperature of the second electrode element, and controlling the applying of the alternating voltage based on the first temperature and the second temperature, as described below.

FIG. 5 depicts one example of hardware that is suitable for this purpose. More specifically, temperature sensors 800 (e.g., thermistors) are positioned in thermal contact with respective electrode elements (for example, dielectric material 70 / layer of metal 76) within each of the electrode assemblies 10a,b. The temperature sensors 800 as shown in FIG. 5 measure respective first and second temperatures (e.g., at a first electrode element in each of the first electrode assembly and second electrode assembly, respectively). Typically, a plurality of such temperature sensors 800 measure respective temperatures at a plurality of electrode elements in each of the first electrode assembly and second electrode assembly, and the controller 822 controls the output of the AC voltage generator 820 based on these temperatures.

While the present invention has been disclosed with reference to certain embodiments, numerous modifications, alterations, and changes to the described embodiments are possible without departing from the scope of the present invention, as defined in the appended claims. Accordingly, it is intended that the present invention not be limited to the described embodiments, but that it has the full scope defined by the language of the following claims.

## Claims

1. An apparatus, comprising:
an electrode layer (20) having:
a plurality of electrode elements (22; 22a-c), each electrode element (22; 22a-c) of the plurality of electrode elements (22; 22a-c) having a skin-facing surface (24; 72) and an opposing outer face (78), wherein at least first and second electrode elements (22a, b) are spaced apart within the electrode layer (20) along a first axis (12); and
at least one filler structure (30; 30a), wherein the at least one filler structure (30; 30a) comprises a first filler structure (30a) having a first filler portion (32a) positioned within a space (34a) between the first and second electrode elements (22a, b), and the at least one filler structure (30; 30a) is configured to provide the electrode layer (20) with a substantially consistent height along the first axis (12) at the height of the outer face (78) of the electrode elements (22; 22a-c);
a layer of anisotropic material (40) having a skin-facing surface (42) and an opposing outwardly-facing surface (44); and
an upper adhesive layer (50) disposed on an outer side (46) of the layer of anisotropic material (40);
wherein the electrode layer (20) is on the outer side (46) of the layer of anisotropic material (40), and at least one electrode element (22; 22a-c) of the plurality of electrode elements (22; 22a-c) is in electrical contact with the outwardly-facing surface (44) of the layer of anisotropic material (40).

2. The apparatus of claim 1, wherein each electrode element (22; 22a-c) of the plurality of electrode elements (22; 22a-c) is in electrical contact with the outwardly-facing surface (44) of the layer of anisotropic material (40).

3. The apparatus of claim 1, wherein each electrode element (22; 22a-c) of the plurality of electrode elements (22; 22a-c) comprises:
a layer of dielectric material (70) having a skin-facing inner face (72) and an opposing outer face (74), optionally the dielectric material is a ceramic material; and
a metallic layer (76) having an inner face (77) and an opposing outer face (78), wherein the metallic layer (76) is disposed on the outer face (74) of the layer of dielectric material (70).

4. The apparatus of claim 1, wherein the plurality of electrode elements (22; 22a-c) further comprises a third electrode element (22c), the second electrode element (22b) is positioned between, and spaced from, the first and third electrode elements (22a, c) along the first axis (12), and the at least one filler structure (30; 30a) further comprises a second filler portion positioned between the second and third electrode elements (22b, c) along the first axis (12).

5. The apparatus of claim 1, wherein the plurality of electrode elements (22; 22a-c) comprises at least two rows of electrode elements (26; 26a, b) that extend along or parallel to a second axis (14) that is perpendicular to the first axis (12), the at least two rows of electrode elements (26; 26a, b) comprises a first row of electrode elements (26a) that includes the first electrode element (22a) and a second row of electrode elements (26b) that includes the second electrode element (22b), and the first filler portion (32a) is positioned between the first and second rows of electrode elements (26a, b) along the first axis (12).

6. The apparatus of claim 5, wherein the plurality of electrode elements (22; 22a-c) comprises at least three rows of electrode elements (26; 26a-c) that extend along or parallel to a second axis (14) that is perpendicular to the first axis (12), the at least three rows of electrode elements (26; 26a-c) comprises a first row of electrode elements (26a) that includes the first electrode element (22a), a second row of electrode elements (26b) that includes the second electrode element (22b) and a third row of electrode elements (26c) that includes the third electrode element (22c), the first filler portion (32a) is positioned between the first and second rows of electrode elements (26a, b), and the second filler portion is positioned between the second and third rows of electrode elements (26b, c), optionally each row of electrode elements (26; 26a-c) comprises three electrode elements (22; 22a-c).

7. The apparatus of claim 5, wherein (i) the first filler portion (32a) and/or the second filler portion is part of a strip of filler structure material that fills the gap between either one or more rows of electrode elements (26) or one or more columns of electrode elements (28), or (ii) the first filler portion (32a) and the second filler portion are part of a shaped layer of filler structure material that fills the gaps between all of the electrode elements (22; 22a-c), having cut-outs (35) as void spaces that receive respective electrode elements (22; 22a-c) of the plurality of electrode elements (22; 22a-c).

8. The apparatus of claim 1, wherein the layer of anisotropic material (40) has a first thermal conductivity in a direction that is perpendicular to a plane of the layer of anisotropic material (40), and thermal conductivity of the layer of anisotropic material (40) in directions that are parallel to the plane of the layer of anisotropic material (40) is more than two times higher than the first thermal conductivity.

9. The apparatus of claim 1, wherein the layer of anisotropic material (40) has a first resistance in a direction that is perpendicular to a plane of the layer of anisotropic material (40), and resistance of the layer of anisotropic material (40) in directions that are parallel to the plane of the layer of anisotropic material (40) is less than half the first resistance.

10. The apparatus of claim 1, wherein the anisotropic material comprises a synthetic graphite, or the layer of anisotropic material (40) is or comprises a layer of pyrolytic graphite, graphitized polymer film or graphite foil made from compressed high-purity exfoliated mineral graphite.

11. The apparatus of claim 1, further comprising:
a skin contact structure (60) having an adhesive layer comprising a conductive adhesive composite, wherein the skin contact structure (60) is disposed on an inner skin-facing side (48) of the layer of anisotropic material (40) and configured to contact skin of a subject, optionally the skin contact structure (60) comprises:
an outer adhesive layer (62) comprising a conductive adhesive composite;
a skin-facing inner adhesive layer (64) comprising a conductive adhesive composite; and
a substrate (66) positioned between the inner and outer adhesive layers (64, 62);
optionally the conductive adhesive composite of the inner and outer adhesive layers (64, 62) each, individually, comprises a dielectric material and conductive particles dispersed within the dielectric material.

12. The apparatus of claim 1, wherein the at least one filler structure (30; 30a) is or comprises a foam, a gel or a hydrocolloid.

13. The apparatus of claim 1, wherein the at least one filler structure (30; 30a) comprises a medication.

14. The apparatus of claim 1, wherein the first filler portion (32a) has a shape that is complementary to a shape of the space (34a) between the first and second electrode elements (22a, b).

15. The apparatus of claim 1, wherein the upper adhesive layer (50) comprises a conductive adhesive composite.

## Patentansprüche

1. Apparat, umfassend:
eine Elektrodenschicht (20) mit:
eine Vielzahl von Elektrodenelementen (22; 22a-c), wobei jedes Elektrodenelement (22; 22a-c) der Vielzahl von Elektrodenelementen (22; 22a-c) eine der Haut zugewandte Oberfläche (24; 72) und eine gegenüberliegende Außenfläche (78) aufweist, wobei zumindest ein erstes und ein zweites Elektrodenelement (22a, b) innerhalb der Elektrodenschicht (20) entlang einer ersten Achse (12) voneinander beabstandet sind; und
mindestens eine Füllstruktur (30; 30a), wobei die mindestens eine Füllstruktur (30; 30a) eine erste Füllstruktur (30a) mit einem ersten Abschnitt (32a) umfasst, der in einem Raum (34a) zwischen dem ersten und dem zweiten Elektrodenelement (22a, b) positioniert ist, und die mindestens eine Füllstruktur (30; 30a) so konfiguriert ist, dass sie der Elektrodenschicht (20) eine im Wesentlichen gleichmäßige Höhe entlang der ersten Achse (12) in Höhe der Außenfläche (78) der Elektrodenelemente (22; 22a-c) verleiht;
eine Schicht aus anisotropem Material (40) mit einer der Haut zugewandten Oberfläche (42) und einer gegenüberliegenden, nach außen gerichteten Oberfläche (44); und
eine obere Klebeschicht (50), die auf einer Außenseite (46) der Schicht aus anisotropem Material (40) angeordnet ist;
wobei sich die Elektrodenschicht (20) auf der Außenseite (46) der Schicht aus anisotropem Material (40) befindet und mindestens ein Elektrodenelement (22; 22a-c) der mehreren Elektrodenelemente (22; 22a-c) in elektrischem Kontakt mit der nach außen gerichteten Oberfläche (44) der Schicht aus anisotropem Material (40) in elektrischem Kontakt steht.

2. Apparat nach Anspruch 1, wobei jedes Elektrodenelement (22; 22a-c) der mehreren Elektrodenelemente (22; 22a-c) in elektrischem Kontakt mit der nach außen gerichteten Oberfläche (44) der Schicht aus anisotropem Material (40) steht.

3. Apparat nach Anspruch 1, wobei jedes Elektrodenelement (22; 22a-c) der mehreren Elektrodenelemente (22; 22a-c) umfasst:
eine Schicht aus dielektrischem Material (70) mit einer der Haut zugewandten Innenfläche (72) und einer gegenüberliegenden Außenfläche (74), wobei das dielektrische Material optional ein Keramikmaterial ist; und
eine Metallschicht (76) mit einer Innenseite (77) und einer gegenüberliegenden Außenseite (78), wobei die Metallschicht (76) auf der Außenseite (74) der Schicht aus dielektrischem Material (70) angeordnet ist.

4. Apparat nach Anspruch 1, wobei die mehrere Elektrodenelemente (22; 22a-c) ferner ein drittes Elektrodenelement (22c) umfassen, das zweite Elektrodenelement (22b) zwischen dem ersten und dem dritten Elektrodenelement (22a, c) entlang der ersten Achse (12) positioniert und von diesen beabstandet ist und die mindestens eine Füllstruktur (30; 30a) ferner einen zweiten Abschnitt umfasst, der zwischen dem zweiten und dem dritten Elektrodenelement (22b, c) entlang der ersten Achse (12) positioniert ist.

5. Apparat nach Anspruch 1, wobei die Vielzahl von Elektrodenelementen (22; 22a-c) mindestens zwei Reihen von Elektrodenelementen (26; 26a, b) umfasst, die sich entlang oder parallel zu einer zweiten Achse (14) erstrecken, die senkrecht zur ersten Achse (12) ist, wobei die mindestens zwei Reihen von Elektrodenelementen (26; 26a, b) eine erste Reihe von Elektrodenelementen (26a) umfasst, die das erste Elektrodenelement (22a) enthält, und eine zweite Reihe von Elektrodenelementen (26b), die das zweite Elektrodenelement (22b) enthält, und der erste Abschnitt (32a) zwischen der ersten und der zweiten Reihe von Elektrodenelementen (26a, b) entlang der ersten Achse (12) positioniert ist.

6. Apparat nach Anspruch 5, wobei die Vielzahl von Elektrodenelementen (22; 22a-c) mindestens drei Reihen von Elektrodenelementen (26; 26a-c) umfasst, die sich entlang oder parallel zu einer zweiten Achse (14) erstrecken, die senkrecht zur ersten Achse (12) ist, wobei die mindestens drei Reihen von Elektrodenelementen (26; 26a-c) eine erste Reihe von Elektrodenelementen (26a) umfasst, die das erste Elektrodenelement (22a) enthält, eine zweite Reihe von Elektrodenelementen (26b), die das zweite Elektrodenelement (22b) enthält, und eine dritte Reihe von Elektrodenelementen (26c), die das dritte Elektrodenelement (22c) enthält, der erste Abschnitt (32a) zwischen der ersten und der zweiten Reihe von Elektrodenelementen (26a, b) positioniert ist und der zweite Abschnitt zwischen der zweiten und der dritten Reihe von Elektrodenelementen (26b, c) positioniert ist, wobei optional jede Reihe von Elektrodenelementen (26; 26a-c) drei Elektrodenelemente (22; 22a-c) umfasst.

7. Apparat nach Anspruch 5, wobei (i) der erste Füllabschnitt (32a) und/oder der zweite Füllabschnitt Teil eines Streifens aus Füllstrukturmaterial ist, der den Spalt zwischen entweder einer oder mehreren Reihen von Elektrodenelementen (26) oder einer oder mehreren Spalten von Elektrodenelementen (28) ausfüllt, oder (ii) der erste Füllabschnitt (32a) und der zweite Füllabschnitt Teil einer geformten Schicht aus Füllstrukturmaterial sind, welche die Spalten zwischen allen Elektrodenelementen (22; 22a-c) ausfüllt und Ausschnitte (35) als Hohlräume aufweist, die jeweilige Elektrodenelemente (22; 22a-c) der Vielzahl von Elektrodenelementen (22; 22a-c) aufnehmen.

8. Apparat nach Anspruch 1, wobei die Schicht aus anisotropem Material (40) eine erste Wärmeleitfähigkeit in einer Richtung aufweist, die senkrecht zu einer Ebene der Schicht aus anisotropem Material (40) ist, und die Wärmeleitfähigkeit der Schicht aus anisotropem Material (40) in Richtungen, die parallel zu der Ebene der Schicht aus anisotropem Material (40) sind, mehr als doppelt so hoch ist wie die erste Wärmeleitfähigkeit.

9. Apparat nach Anspruch 1, wobei die Schicht aus anisotropem Material (40) einen ersten Widerstand in einer Richtung aufweist, die senkrecht zu einer Ebene der Schicht aus anisotropem Material (40) ist, und der Widerstand der Schicht aus anisotropem Material (40) in Richtungen, die parallel zu der Ebene der Schicht aus anisotropem Material (40) sind, weniger als die Hälfte des ersten Widerstands beträgt.

10. Apparat nach Anspruch 1, wobei das anisotrope Material synthetischen Graphit umfasst oder die Schicht aus anisotropem Material (40) eine Schicht aus pyrolytischem Graphit, graphitisiertem Polymerfilm oder Graphitfolie aus komprimiertem, hochreinem, abgeblättertem Mineralgraphit ist oder umfasst.

11. Apparat nach Anspruch 1, ferner umfassend:
eine Hautkontaktstruktur (60) mit einer Klebeschicht, die einen leitfähigen Klebstoffverbundstoff umfasst, wobei die Hautkontaktstruktur (60) auf einer inneren, der Haut zugewandten Seite (48) der Schicht aus anisotropem Material (40) angeordnet und so konfiguriert ist, dass sie mit der Haut eines Subjekts in Kontakt kommt, wobei die Hautkontaktstruktur (60) optional Folgendes umfasst:
eine äußere Klebeschicht (62), die einen leitfähigen Klebstoffverbundstoff umfasst; eine der Haut zugewandte innere Klebeschicht (64), die einen leitfähigen Klebstoffverbundstoff umfasst; und
ein Substrat (66), das zwischen der inneren und der äußeren Klebeschicht (64, 62) angeordnet ist;
optional umfasst der leitfähige Klebstoffverbundstoff der inneren und äußeren Klebeschichten (64, 62) jeweils einzeln ein dielektrisches Material und leitfähige Partikel, die in dem dielektrischen Material dispergiert sind.

12. Apparat nach Anspruch 1, wobei die mindestens eine Füllstoffstruktur (30; 30a) ein Schaum, ein Gel oder ein Hydrokolloid ist oder umfasst.

13. Apparat nach Anspruch 1, wobei die mindestens eine Füllstruktur (30; 30a) ein Medikament umfasst.

14. Apparat nach Anspruch 1, wobei der erste Füllstoffabschnitt (32a) eine Form aufweist, die komplementär zu einer Form des Raums (34a) zwischen dem ersten und dem zweiten Elektrodenelement (22a, b) ist.

15. Apparat nach Anspruch 1, wobei die obere Klebeschicht (50) einen leitfähigen Klebstoffverbundstoff umfasst.

## Revendications

1. Appareil, comprenant :
une couche d'électrodes (20) comportant :
une pluralité d'éléments d'électrode (22 ; 22a-c), chaque élément d'électrode (22 ; 22a-c) de la pluralité d'éléments d'électrode (22 ; 22a-c) ayant une surface tournée vers la peau (24 ; 72) et une face extérieure opposée (78), dans lequel au moins des premier et deuxième éléments d'électrode (22a, b) sont espacés l'un de l'autre dans la couche d'électrode (20) le long d'un premier axe (12) ; et
au moins une structure de remplissage (30; 30a), dans laquelle la au moins une structure de remplissage (30 ; 30a) comprend une première structure de remplissage (30a) ayant une première partie de remplissage (32a) positionnée dans un espace (34a) entre les premier et deuxième éléments d'électrode (22a, b), et la au moins une structure de remplissage (30; 30a) est configurée pour fournir à la couche d'électrode (20) une hauteur sensiblement constante le long du premier axe (12) à la hauteur de la face extérieure (78) des éléments d'électrode (22 ; 22a-c) ;
une couche de matériau anisotrope (40) présentant une surface tournée vers la peau (42) et une surface opposée tournée vers l'extérieur (44) ; et
une couche adhésive supérieure (50) disposée sur un côté extérieur (46) de la couche de matériau anisotrope (40) ;
dans lequel la couche d'électrode (20) se trouve sur le côté extérieur (46) de la couche de matériau anisotrope (40), et au moins un élément d'électrode (22 ; 22a-c) de la pluralité d'éléments d'électrode (22 ; 22a-c) est en contact électrique avec la surface tournée vers l'extérieur (44) de la couche de matériau anisotrope (40).

2. Dispositif selon la revendication 1, dans lequel chaque élément d'électrode (22 ; 22a-c) de la pluralité d'éléments d'électrode (22 ; 22a-c) est en contact électrique avec la surface tournée vers l'extérieur (44) de la couche de matériau anisotrope (40).

3. Appareil selon la revendication 1, dans lequel chacun des ensembles d'éléments d'électrode (22 ; 22a-c) de la pluralité d'éléments d'électrode (22 ; 22a-c) comprend :
une couche de matériau diélectrique (70) ayant une face interne (72) tournée vers la peau et une face externe opposée (74), le matériau diélectrique étant éventuellement un matériau céramique ; et
une couche métallique (76) ayant une face interne (77) et une face externe opposée (78), dans laquelle la couche métallique (76) est disposée sur la face externe (74) de la couche de matériau diélectrique (70).

4. Appareil selon la revendication 1, dans lequel la pluralité d'éléments d'électrode (22 ; 22a-c) comprend en outre un troisième élément d'électrode (22c), le deuxième élément d'électrode (22b) est positionné entre les premier et troisième éléments d'électrode (22a, c) et espacé de ceux-ci le long du premier axe (12), et la au moins une structure de remplissage (30 ; 30a) comprend en outre une deuxième partie de remplissage positionnée entre les deuxième et troisième éléments d'électrode (22b, c) le long du premier axe (12).

5. Appareil selon la revendication 1, dans lequel la pluralité d'éléments d'électrode (22 ; 22a-c) comprend au moins deux rangées d'éléments d'électrode (26 ; 26a, b) qui s'étendent le long ou parallèlement à un deuxième axe (14) qui est perpendiculaire au premier axe (12), les au moins deux rangées d'éléments d'électrode (26 ; 26a, b) comprenant une première rangée d'éléments d'électrode (26a) qui comprend le premier élément d'électrode (22a) et une deuxième rangée d'éléments d'électrode (26b) qui comprend le deuxième élément d'électrode (22b), et la première partie de remplissage (32a) étant positionnée entre les première et deuxième rangées d'éléments d'électrode (26a, b) le long du premier axe (12).

6. Appareil selon la revendication 5, dans lequel la pluralité d'éléments d'électrode (22 ; 22a-c) comprend au moins trois rangées d'éléments d'électrode (26 ; 26a-c) qui s'étendent le long ou parallèlement à un deuxième axe (14) qui est perpendiculaire au premier axe (12), les au moins trois rangées d'éléments d'électrode (26 ; 26a-c) comprenant une première rangée d'éléments d'électrode (26a) qui comprend le premier élément d'électrode (22a), une deuxième rangée d'éléments d'électrode (26b) qui comprend le deuxième élément d'électrode (22b) et une troisième rangée d'éléments d'électrode (26c) qui comprend le troisième élément d'électrode (22c), la première partie de remplissage (32a) est positionnée entre les première et deuxième rangées d'éléments d'électrode (26a, b), et la deuxième partie de remplissage est positionnée entre les deuxième et troisième rangées d'éléments d'électrode (26b, c), chaque rangée d'éléments d'électrode (26 ; 26a-c) comprenant éventuellement trois éléments d'électrode (22 ; 22a-c).

7. Appareil selon la revendication 5, dans lequel (i) la première partie de remplissage (32a) et/ou la deuxième partie de remplissage fait partie d'une bande de matériau de structure de remplissage qui comble l'espace entre une ou plusieurs rangées d'éléments d'électrode (26) ou une ou plusieurs rangées d'éléments d'électrode (28), ou (ii) la première partie de remplissage (32a) et la deuxième partie de remplissage font partie d'une couche profilée de matériau de structure de remplissage qui comble les espaces entre tous les éléments d'électrode (22 ; 22a-c), comportant des découpes (35) sous forme d'espaces vides qui reçoivent les éléments d'électrode respectifs (22 ; 22a-c) de la pluralité d'éléments d'électrode (22 ; 22a-c).

8. Appareil selon la revendication 1, dans lequel la couche de matériau anisotrope (40) présente une première conductivité thermique dans une direction qui est perpendiculaire au plan de la couche de matériau anisotrope (40) et conductivité thermique de la couche de matériau anisotrope (40) dans des directions qui sont parallèles au plan de la couche de matériau anisotrope (40) est plus de deux fois supérieure à la première conductivité thermique.

9. Appareil selon la revendication 1, dans lequel la couche de matériau anisotrope (40) présente une première résistance dans une direction qui est perpendiculaire à un plan de la couche de matériau anisotrope (40), et la résistance de la couche de matériau anisotrope (40) dans des directions qui sont parallèles au plan de la couche de matériau anisotrope (40) est inférieure à la moitié de la première résistance.

10. Appareil selon la revendication 1, dans lequel le matériau anisotrope comprend un graphite synthétique, ou la couche de matériau anisotrope (40) est ou comprend une couche de graphite pyrolytique, un film polymère graphité ou une feuille de graphite fabriquée à partir de graphite minéral exfolié de haute pureté comprimé.

11. Appareil selon la revendication 1, comprenant en outre :
une structure de contact avec la peau (60) comportant une couche adhésive comprenant un composite adhésif conducteur, dans lequel la structure de contact avec la peau (60) est disposée sur un côté intérieur (48) de la couche de matériau anisotrope (40) faisant face à la peau et configurée pour entrer en contact avec la peau d'un sujet, la structure de contact avec la peau (60) comprenant éventuellement :
une couche adhésive externe (62) comprenant un composite adhésif conducteur ; une couche adhésive interne (64) tournée vers la peau comprenant un composite adhésif conducteur ; et
un substrat (66) positionné entre les couches adhésives interne et externe (64, 62) ;
éventuellement, le composite adhésif conducteur des couches adhésives interne et externe (64, 62) comprend chacun, individuellement, un matériau diélectrique et des particules conductrices dispersées dans le matériau diélectrique.

12. Appareil selon la revendication 1, dans lequel la au moins une structure de remplissage (30; 30a) est ou comprend une mousse, un gel ou un hydrocolloïde.

13. Dispositif selon la revendication 1, dans lequel la au moins une structure de remplissage (30 ; 30a) comprend un médicament.

14. Dispositif selon la revendication 1, dans lequel la première partie de remplissage (32a) a une forme complémentaire à la forme de l'espace (34a) entre les premier et deuxième éléments d'électrode (22a, b).

15. Dispositif selon la revendication 1, dans lequel la couche adhésive supérieure (50) comprend un composite adhésif conducteur.
